# EUROPEAN PATENT APPLICATION

(11) **EP 1 273 307 A1**
(43) Date of publication of application: **08.01.2003**
(21) Application number: 01917586.8
(22) Date of filing: 29.03.2001
(51) Int. Cl.: A61K 47/30, A61K 9/16, A61K 9/52, A61K 45/00, A61K 45/06, A61K 31/235, A61P 1/12, A61P 3/06

(54) **PREPARATIONS FOR PREVENTING BILE ACID DIARRHEA**

(30) Priority: 10.04.2000 JP 2000107431
(71) Applicant: SHIONOGI & CO., LTD., Osaka-shi, Osaka 541-0045 (JP)
(72) Inventor: MASUDA, Kazuyoshi, c/o Shionogi & Co., Ltd., Osaka-shi, Osaka 553-0002 (JP); SUGITA, Katsuji c/o Shionogi & Co., Ltd., Osaka-shi, Osaka 553-0002 (JP); HAYAKAWA, Hiroshi, c/o Shionogi & Co., Ltd., Settsu-shi, Osaka 566-0022 (JP); SYODAI, Hidekazu, c/o Shionogi & Co., Ltd., Amagasaki-shi, Hyogo 660-0813 (JP); SUZUKI, Yusuke, c/o Shionogi & Co., Ltd., Settsu-shi, Osaka 566-0022 (JP)
(74) Representative: Baverstock, Michael George Douglas
(86) International application number: JP0102618
(87) International publication number: WO01076635

(57) **Abstract**

Preparations for preventing bile acid diarrhea which comprise containing a bile acid adsorbent coated with a polymer so as to allow the release thereof around an area from the lower part of the small intestine to the cecum; and pharmaceutical compositions comprising a combination of bile acid re-absorption inhibitors with the above preparations for preventing bile acid diarrhea (e.g., antihyperlipidaemic agent).

## Description

### Technical Field

The present invention relates to a preparation for preventing bile acid diarrhea, a pharmaceutical composition which comprises a combination of a bile acid re-absorption inhibitor and the preparation for preventing diarrhea, and the like.

### Background Art

It was reported by LRC-CPPT (Lipid Research Clinics Coronary Primary Prevention Trial), U.S.A, in 1984 that the crisis rate of coronary artery disease can be reduced by the treatment of hypercholesterolemia using a bile acid excretion-accelerating agent. Since then, various hypercholesterolemia-treating agents based on the pharmacological mechanism have been developed. Among them, bile acid re-absorption inhibitors, such as lignan analogs (JP Patent Publication (A) 1993/310634, USP 5,420,333) or glucuronic acid conjugates thereof (JP Patent Publication (A) 1997/241206) etc.), are known to inhibit the re-absorption of bile acid from the small intest by the inhibition of bile acid transporter (BAT), so as to lower the concentration of LDL-cholesterol. However, the inhibition of the re-absorption of bile acid leads to a large quantity of flow of the bile acid into the large intestine, causing the increase of the bile acid concentration therein. In such a case, there is a concern that bile acid diarrhea may be induced in the large intestine depending on the type of patients, health status thereof, or the like.

On the contrary, bile acid adsorbents, such as anion-exchange resins represented by cholestyramine, are known to inhibit the enterohepatic circulation of bile acid by absorbing bile acid in the intestinal tract to excrete it into excrement, whereby to lower the LDL-cholesterol concentration. For example, JP Patent Publication (A) 1988/152321 discloses a preparation of cholestyramine for sustained-release inside gastrointestinal, wherein the surface thereof is coated so as to stabilize the preparation until it reaches the top of the small intestine for the purpose of maximizing the efficacy of cholestyramine.

Cholestyramine has been reported to possess an inhibitory effect on bile acid diarrhea (AM. J. Med. Sci., 84-88, *255* (1968), Gut 531-535 *18* (1977) etc.). However, a preparation of cholestyramine has not been reported, which is designed to release it selectively at a specific site inside gastrointestinal, especially, around a area from the lower part of the small intestine to the cecum in order to more effectively inhibit bile acid diarrhea.

Accordingly, it has been targeted to develop a preparation capable of effectively inhibiting bile acid diarrhea, of which inducement is concerned upon oral administration of an bile acid re-absorption inhibitor or the like. Further, in the development of an antihyperlipidaemic agent based on the inhibition of bile acid re-absorption, it has been desired to provide a preparation of bile acid re-absorption inhibitor which can exhibit the pharmacological effect without inducing bile acid diarrhea.

### Disclosure of Invention

The present inventors have intensively studied to find that the inducement of bile acid diarrhea can be effectively prevented by coating the surface of an bile acid adsorbent for administration so as to allow a selective release thereof at a specific site inside gastrointestinal, especially, around an area from the lower part of the small intestine to the cecum, thereby accomplishing the present invention shown below.
1. A preparation for preventing bile acid diarrhea, which comprises containing a bile acid adsorbent coated with a polymer so as to allow the release of the bile acid adsorbent around an area from the lower part of the small intestine to the cecum.
2. The preparation for preventing diarrhea according to above 1, wherein the polymer is a water-insoluble polymer.
3. The preparation for preventing diarrhea according to above 2, wherein the water-insoluble polymer is an enteric polymer or a pH-independent polymer.
4. The preparation for preventing diarrhea according to above 3, wherein the enteric polymer is hydroxypropylmethylcellulose acetate succinate, hydroxypropylmethylcellulose phthalate or a methacrylic acid copolymer, and the pH-independent polymer is ethyl cellulose or an aminoalkylmethacrylic acid copolymer.
5. The preparation for preventing diarrhea according to above 1, wherein an isolation layer is placed between the bile acid adsorbent and the polymer.
6. The preparation for preventing diarrhea according to above 5, wherein the main ingredient of the isolation layer is phosphate or sulfate.
7. The preparation for preventing diarrhea according to above 1, which is a granule.
8. A capsule which contains the granules of above 7.
9. A preparation for preventing bile acid diarrhea, which comprises a capsule containing a bile acid adsorbent, wherein the surface of the capsule is coated with a polymer so as to allow the release of the bile acid adsorbent around an area from the lower part of the small intestine to the cecum .
10. A pharmaceutical composition, which comprises a combination of a bile acid re-absorption inhibitor and the preparation for preventing diarrhea of any one of above 1 to 9.
11. The pharmaceutical composition according to above 10, wherein the form of the bile acid re-absorption inhibitor is a granule, powder or a capsule.
12. The pharmaceutical composition according to above 10, which comprises a combination of granules or powder, each containing the bile acid re-absorption inhibitor, and the preparation for preventing diarrhea of above 7.
13. The pharmaceutical composition according to above 10, which is a capsule containing the bile acid re-absorption inhibitor and the bile acid adsorbent, wherein the surface of the capsule is coated with a polymer so as to allow the release of the bile acid adsorbent around an area from the lower part of the small intestine to the cecum.
14. The pharmaceutical composition according to any one of above 10 to 13,
   wherein the bile acid re-absorption inhibitor is absorbed in an area of the upper and middle parts of the small intestine.
15. The pharmaceutical composition according to above 14, wherein the bile acid adsorbent is released around an area from the lower part of the small intestine to the cecum within several hours, after the absorption of the bile acid re-absorption inhibitor.
16. The pharmaceutical composition according to above 14, wherein the bile acid adsorbent is released around an area from the lower part of the small intestine to the cecum at three to four fours after the absorbance of the bile acid re-absorption inhibitor.
17. The pharmaceutical composition according to above 10 for use as an antihyperlipidaemic agent.
18. The pharmaceutical composition according to any one of above 10 to 17,
   wherein the bile acid re-absorption inhibitor is methyl 1-(3,4-dimethoxyphenyl)-3-(3-ethylvaleryl)-4-hydroxy-6, 7, 8-trimethoxy-2-naphthoate or a glucuronic acid conjugate thereof.
19. Use of a bile acid adsorbent which is coated with a polymer so as to allow the release thereof, for preventing bile acid diarrhea upon oral administration of a bile acid re-absorption inhibitor.
20. Use according to above 19, wherein the bile acid adsorbent is released around an area from the lower part of the small intestine to the cecum within several hours, after the absorption of the bile acid re-absorption inhibitor in an area of the upper and middle parts of the small intestine.
21. A method for preventing bile acid diarrhea upon oral administration of a bile acid re-absorption inhibitor, which comprises oral administration of the bile acid re-absorption inhibitor and a bile acid adsorbent coated with a polymer so as to allow the release of the bile acid adsorbent around an area from the lower part of the small intestine to the cecum, at the same time or a certain interval.
22. The method for preventing bile acid diarrhea according to above 21, wherein the bile acid adsorbent is released around an area from the lower part of the small intestine to the cecum within several hours, after the absorbance of the bile acid re-absorption inhibitor in an area of the upper and middle parts of the small intestine.
23. A method for preventing or treating hyperlipemia, which comprises oral administration of the bile acid re-absorption inhibitor and a bile acid adsorbent coated with a polymer so as to allow the release of the bile acid adsorbent around an area from the lower part of the small intestine to the cecum, at the same time or a certain interval.

### Brief Description of Drawings

### (Figure 1)

This chart shows volume change-time profiles for CSA-coated granules of Example 1 in the first fluid of the Japanese Pharmacopoeia. The horizontal axis shows time (minute) and the vertical axis shows the swelling rate (%).

### (Figure 2)

This chart shows volume change-time profiles for CSA-coated granules of Example 2 in the first fluid of the Japanese Pharmacopoeia. The horizontal axis shows time (minute) and the vertical axis shows the swelling rate(%).

### Best Mode for Carrying Out the Invention

As a bile acid adsorbent for the present invention, various adsorbents can be used. The examples include basic anion-exchange resins (e.g., Cholestyramine (polystyrene benzyltrimethylammonium chloride), Colestilan (2-methyl-1H-imidazole polymer with (chloromethyl)oxirane), KBS-275 (poly[N,N-dimethyl-N-[1,4-phenyleneether-6-methyl-2-propyl]-N-propylammonium chloride]), HBS-107, unabsorbable aqueous gels (e.g., Colesevelam hydrochloride), and cationic natural polymers (e.g., chitosan)). The bile acid adsorbent can be used as a nuclear particle for coating, and the average particle size is about 10 to 2000µm, and preferably about 100 to 1000 µm. Such a preferable range of the average particle size can make the polymer-coating very easy and control the variance of the gastric emptying rate of the invention preparation.

Preferred examples of the polymer, which is used for selectively release a bile acid adsorbent around an area from the lower part of the small intestine to the cecum, include a water-insoluble polymer, and more preferred are an enteric polymer, a pH-independent polymer, and the like.

Examples of the enteric polymer include hydroxypropylmethylcellulose acetate succinate, hydroxypropylmethylcellulose phthalate, methacrylic acid copolymer (e.g., Eudragit L, Eudragit S). Preferred is hydroxypropylmethylcellulose acetate succinate (HPMCAS), esp., H-type which is usually soluble at pH about 4.5 to 7, and preferably pH about 6 to 7.

Examples of a pH-independent polymer include ethyl cellulose, an aminoalkylmethacrylic acid copolymer (e.g., Eudragit RL, Eudragit RS, Eudragit NE) and preferably, ethyl cellulose (EC). The polymer can be coated on a naked granule so as to be of an optional thickness, thereby allowing a bile acid adsorbent contained therein to selectively release around an area from the lower part of the small intestine to the cecum, without releasing it in the stomach or around the top and middle of the small intestine.

Examples of a method for coating the bile acid adsorbent with the above polymer include various methods well known to skilled persons in the invention, e.g., a fluidized bed method, a rotating fluidized bed method, a side-vented pan or coating pan method, and preferably a fluidized bed method with a Wurster column.

Examples of a solvent for preparing coating solution include e.g., ethanol, dichloromethane, acetone, isopropanol, and water. The above polymer may be dissolved or suspended in a solvent so as to make the final concentration about 0.5 to about 30%, and preferably about 5 to about 15%. If necessary, an optional additive for the usual coating, such as plasticizers (e.g., triethyl citrate, propylene glycol), poders (e.g., talc, titanium oxide), can be added. The coating rate of the above polymer to a bile acid adsorbent, variable depending on the kind of the polymer, is usually about 10 to 100%, preferably about 15 to 75%, and more preferably about 20 to 70%, about 25 to 70%, or about 30 to 70%, by weight.

When the coating rate is excessively low, the bile acid adsorbent inevitably releases around an area from the stomach to the small intestine. In contrast, an excessively high coating rate does not allow the bile acid adsorbent to release sufficiently when the preparation reaches to around an area from the lower part of the small intestine to the cecum. Thus, in such cases, bile acid is not effectively adsorbed around an area from the lower part of the small intestine to the cecum, and as a result, diarrhea can not be prevented.

In case that the above coating causes a trouble such as insolublization of the above polymer due to an interaction between the polymer and an bile acid adsorbent, an isolation layer may be inserted between the polymer and the bile acid adsorbent. Examples of the main ingredient of the isolation layer include phosphates (e.g., calcium hydrogen phosphate) and sulfates (e.g., calcium sulphate). A coating solution for forming the isolation layer can be prepared by dissolving such an ingredient, if necessary together with a binding agent (e.g., hydroxypropylcellulose (HPC)) in an oraganic solvent mentioned above. The coating rate of a coating solution to a bile acid adsorbent is usually about 5 to 30%, and preferably about 10 to 20% by weight. Within such preferable coating rates, the interaction between a polymer and a bile acid adsorbent can be sufficiently controlled without excessively inhibiting the release of a bile acid adsorbent.

The form of the invention preparation for preventing diarrhea prepared by coating a bile acid adsorbent with the above polymer is, not necessarily limited thereto, preferably a granule or a capsule containing the granule. Examples of an ingredient for the coating include gelatin or hydroxypropylmethylcellulose. The content ratio of a polymer-coated bile acid adsorbent is usually about 1 to 100%, and preferably about 5 to 50%, per the total weight of the ingredients contained in the capsule.

As another embodiment of the present invention, provided is a preparation for preventing bile acid diarrhea, which comprises that the surface of a capsule is coated with a polymer so as to release a bile acid adsorbent around an area from the lower part of the small intestine to the cecum. As the polymer or capsule, the above mentioned examples can be used, and the coating ratio of a polymer to the capsule containing a bile acid adsorbent is usually about 10 to 100% by weight.

The above preparation for preventing diarrhea may contain an optional additive used for a pharmaceutical composition or a food, such as a binder, an excipient, a disintegrator, and a dispersant. Preferably, the preparation for preventing diarrhea can be orally administered at the same time as or at an appropriate interval after taking a pharmaceutical composition or food.

The mechanism of inducement of bile acid diarrhea at the large bowel is considered as follows. First, bile acid is excessively secreted and flowed into the large bowel due to inhibition of re-absorption thereof at the small intestine or the like. Second, the main ingredient of bile acid is converted into deoxycholic acid (DCA) by inner-intestinal bacteria, resulting in the increase of water secretion in the appendix. Further, a recent study reported that the amount of administered granules at a lower part of the small intestine reached to the maximun at 1 hour after the administration to fasted beagle dogs (Journal of Pharmaceutical Sciences and Technology, Japan 59, 148-155, 1999). Accordingly, in a case that all of the administered granules burst within the time of 1 hour after the administration, until which the amount of the granules at a lower part of the small intestine reaches to the maximun, or in another case that the granules hardly burst even at the same part, the prevention effect on bile acid diarrhea of beagle dogs is not expected. This is because such granules can not release the contained bile acid adsorbent effectively around the cecum.

On the other hand, a preparation of the present invention can effectively prevent bile acid diarrhea by controling the release site of a bile acid re-absorption inhibitor inside gastrointestinal. As shown in Tests 1 and 2 using beagle dogs, bile acid diarrhea can effectively be prevented by CSA granules: the enteric coated granule of Example 1 and ethylcellulose (EC) coated granules C and D of Example 2, of which time to reach the 50% burst at the 1^{st} fluid (about pH 1.2) is around 40 to 80 minutes. These results suggest that a present preparation prevents bile acid diarrhea based on the mechanizum that it releases a contained bile acid re-absorption inhibitor selectively around an area from the lower part of the small intestine to the cecum, with hardly bursting at the stomach or around a lower part of the small intestine.

In addition, it is generally reported that the length of the small intestine of human, distance from the pylorus to the cecum, is about 6.3 to 7.3 m and that of a dog is about 3.5 times longer than its body length. Of the beagle dogs used in the tests mentioned below, the length of the small intestine is speculated about 2.5 m. Further, the small intestine - pass time of granules in beagle dogs has been reported as less than 1 hour, 1/3 to 1/4 times of that in human, which is supposed as being almost correlated with the length of the small intestine. These considerations suggest that in order to practically exhibit the effect of preventing bile acid diarrhea even in human, preferably used is a preparation of the present invention wherein the time to reach the coate-bursting is longer than that of the aforementioned preparation particularly effective for dogs. Such a preferable preparation is E type of Example 2 for example.

As mentioned above, the present preparation is effective against the bile acid diarrhea of mammals, esp., human. In a case that a bile acid adsorbent itself is orally administered to human as an antihyperlipidaemic agent, the usual dose per one unit for an adult is as large as about 100 to 500 mg/kg. In contrast, when such a bile acid adsorbent is administered as being contained in the present preparation for the purpose of preventing diarrhea, the usual dose per one unit for an adult is as small as about 0.1 to 200 mg/kg, and preferably about 2 to 100 mg/kg.

Further, the above preparation for preventing bile acid diarrhea may be used in combination with a bile acid re-absorption inhibitor. The bile acid re-absorption inhibitor, usually useful as an antihyperlipidaemic agent by reducing cholesterol as mentioned above, may be accompanied by a side effect of inducing bile acid diarrhea. However, such diarrhea can effectively be prevented in combination with the present preparation for preventing bile acid diarrhea, whereby to provide a safe pharmaceutical composition without the side effect.

Examples of the bile acid re-absorption inhibitor used for the present invention include, not limited thereto, lignan analogs (JP Patent Publication (A) 1993/310634, USP 5,420,333). Among them, preferred is Example 1 compound. methyl 1-(3,4-dimethoxyphenyl)-3-(3-ethylvaleryl)-4-hydroxy-6,7,8-trimethoxy-2-naphthoate). Another preferable compound is a glucuronic acid conjugate thereof (JP Patent Publication (A) 1997/241206), especially, Example 7 compound 1A-a: [1-0-{4-(3,4-dimethoxyphenyl)-2-(3-ethylpentanoyl)-5,6,7-trimethoxy-3-(methoxycarbonyl) naphthalene-1-yl}-β-D-glucopyranoside]uronic acid). Another preferable compound is GW-264 (WO96/05188), GW-577 (7-bromo-3-butyl-3-ethyl-8-hydroxy-5-phenyl-1,5-benzothiazepine-1,1-dioxide,. WO96/16051), SC-70112 (WO97/33882) or the like. The other examples include one described in WO94/18183, WO94/18184, WO98/40375, WO 96/844484, JP Patent Publication (A) 1998/278568, JP Patent Publication (A) 1998/72371, or WO95/22348. These bile acid re-absorption inhibitors are usually used for oral administration and the dose for an adult is about 0.01 to 50 mg/kg, and preferably about 0.1 to 30 mg/kg.

Examples of a pharmaceutical composition, which comprises a combination of the above bile acid re-absorption inhibitor and a preparation for preventing diarrhea, include, not limited thereto, various formulations capable of fully exhibiting the pharmacological effect without diarrhea. Preferred examples include a combination wherein a bile acid re-absorption inhibitor is a granule, powder, or capsule, and a preparation for preventing diarrhea is granules or a capsule containing the granules. In the pharmaceutical composition, a bile acid re-absorption inhibitor and a preparation for preventing diarrhea may be contacted each other in single package, or they may be separately packed for non-contact. In the case of the single package, the pharmaceutical composition is preferably complex granules containing each granule or a capsule containing the complex granules. Another single package composition may be that wherein the surface of a preparation for preventing diarrhea is coated with a bile acid re-absorption inhibitor. In the case of the separate package, the pharmaceutical composition preferably comprises a package containing granules or powder of a bile acid re-absorption inhibitor and a package containing granules of a preparation for preventing diarrhea.

In another embodiment, the present invention provides a capsule containing a bile acid re-absorption inhibitor and a bile acid adsorbent, wherein the surface of the capsule is coated with a polymer so as to release the bile acid adsorbent around an area from the lower part of the small intestine to the cecum. Examples of the polymer and capsule include the aforementioned types and the method for preparing coating solution and coating can may be according to the methods descrived above.

In the above pharmaceutical composition, it is dispensable to use a bile acid adsorbent in an amount necessary for fully lowering cholesterol. However, the composition may exhibit such a cholesterol-lowering effect without causing any problem. The use ratio of a bile acid re-absorption inhibitor and a bile acid adsorbent is usually about 1:0.01 to 1:500, preferably about 1:0.5 to 1:200, more preferably about 1:1 to 1:50, and most preferably about 1:1 to 1:10.

A bile acid re-absorption inhibitor is absorbed around an upper to middle area of the small intestine within several hours after oral administration. After the absorption of the bile acid re-absorption inhibitor, a bile acid adsorbent is released within several hours, esp. 3 to 4 hours later thereof in human, around an area from the lower part of the small intestine to the cecum, resulting in adsorbing the bile acid which is released inside the intestinal tract by the effect of the bile acid re-absorption inhibitor, whereby to prevent the inducement of diarrhea.

Further, the present invention provides the following inventions:
use of a bile acid adsorbent coated with the above polymer for preventing bile acid diarrhea caused after oral administration of a bile acid re-absorption inhibitor,
a method for preventing bile acid diarrhea which comprises orally administring such a coated bile acid adsorbent upon administration of a bile acid re-absorption inhibitor, and a method for treating or preventing hyperlipemia which comprises the same.

The kind or use amount of a bile acid re-absorption inhibitor, bile acid adsorbent, polymer or the like is the same as mentioned above. A bile acid adsorbent may be preferably administered, not limited thereto, at the same time as or at certain interval (e.g., 30 min to 6 hours) with that of a bile acid re-absorption inhibitor.

Examples of the present invention are shown below.

As a bile acid adsorbent, used was Cholestyramine resin (DOWEX 1 × 2 16-100 mesh; DOWEX company) with an average granule size of about 500µm. Coated granules were prepared with a fluid bed granulator with a Wurster column by using Cholestyramine resin as nuclear particles. Cholestyramine (CSA) resin, obtained as being wetness (initial water content: about 70%, and average granule size: about 700µm), was dried with a ventilation-type dryer (50°C, 1 h) to use it as dry beads. The coat permanency of the coated granules was evaluated by soak test*.
(_{*})Soak test: To an experimental graduate of 10 mL containing 2 mL of coated granules, was added the first fluid of the Japanese Pharmacopoeia (pH about 1.2) to the full, then the swelling rate, a volume change vs time of a CSA resin, was measured. The state of the coat was observed by SEM (Scanning Electron Microscopy) and the relation of the swelling rate vs the bursting rate of the coat was examined.

### (Example 1)

### Preparation of enteric-coated cholestyramine (CSA) preparation (pH-dependent type)

As an enteric layer, used was HPMCAS-HF (Shin-Etsu Chemical Co., Ltd.) having a dissolution pH of 6.5 or more. Enteric-coated granules were prepared by using a coating solution containing HPMCAS-HF / triethyl citrate /(EtOH/CH₂Cl₂, 7:3 v/v) ≒ 7 / 1 / 92 wt %. For the purpose of an interaction between a group of "-COC₂H₄COOH" of HPMCAS and trimethylamine of CSA, an isolation layer was inserted between the enteric layer and CSA. The isolation layer was coated with a solution containing calcium hydrogen phosphate / HPC-SL / EtOH (7 / 3 / 90 wt %). The composition of the obtained granules were shown below.

**(Table 1)**

| Ingredient | Content(%) |
|---|---|
| DOWEX 1×2 16-100 mesh | 69.4 |
| Sieved anhydrous dibasic calcium phosphate | 6.6 |
| HPC-SL | 2.8 |
| HPMCAS-HF | 19.1 |
| Triethyl citrate | 2.1 |
| | 100.0 |

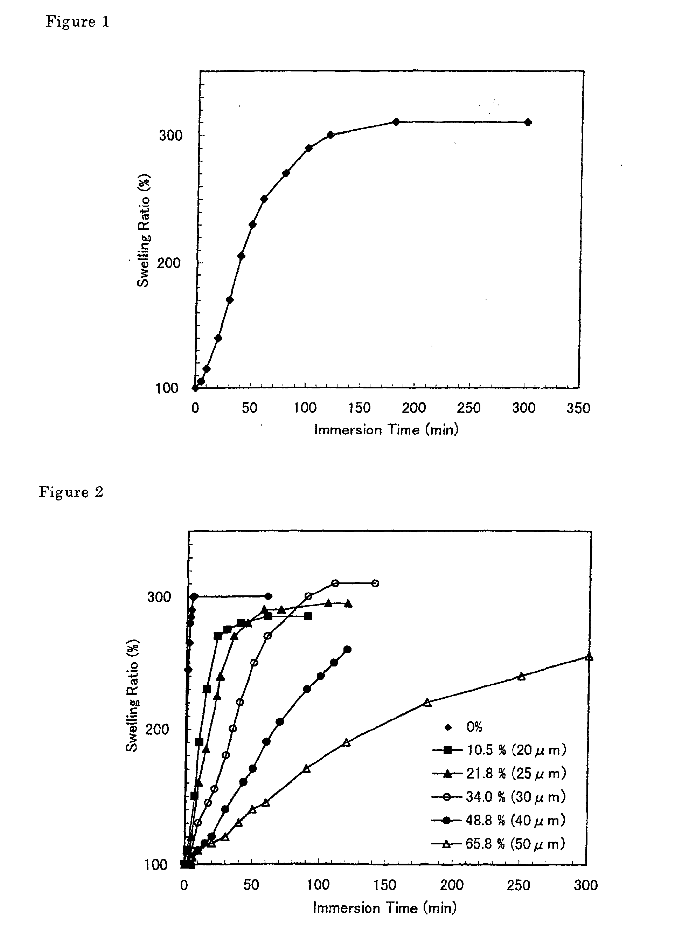

The isolation layer and the enteric coating layer were each confirmed as being a uniform coating layer with a content of 13.6% (coat wideness = about 10µm) and 30.5% (coat wideness = about 30µm), respectively, per the nuclear particle by SEM detection. The result of soak test is shown in Figure 1. The swelling rate was 170% at 30 min, 250% at 1 hr and 300% at 2 hr. By SEM detection, the progress of burst of the coating layer was confirmed as about 20% at 30 min, about 80% at 1 hr, and 100% at 2 hr.

### (Example 2)

### Preparation of ethyl cellulose-coated cholestyramine (CSA) preparation (a type controlled by coat wideness

Five kinds of ethyl cellulose (EC) granules were prepared by using coating solution of EC/EtOH=5/95 wt %, wherein their coating amounts were varied depending on the coating time. Their compositions are shown below.

**(Table 2)**

| Compositions | A | B | B | D | E |
|---|---|---|---|---|---|
| DOWEX 1×2 16-100mesh | 90.5 | 82.1 | 74.6 | 67.2 | 60.3 |
| ethyl cellulose(EC) | 9.5 | 17.9 | 25.4 | 32.8 | 39.7 |
| | 100.0 | 100.0 | 100.0 | 100.0 | 100.0 |
| Coating rate of EC layer(%) | 10.5 | 21.8 | 34.0 | 48.8 | 65.8 |

Each coating layer was confirmed by SEM detection as being uniformly prepared. In the soak test, 50% burst-resistant time (estimated time that 50% of coated film remain without bursting) is deemed as a time when the swelling rate is 220%, 50% burst-resistant time of the present EC granules were prolonged depending on the increase of the coating amount (Fig. 2). In particular, 50% burst-resistant time was inferred as 1.3 hr when the coating amount was 48.8% (coat wideness = about 40µm) and 3.0 hr when the coating amount was 65.8% (coat wideness = about 50µm), which was accorded with the results of SEM detection wherein a half amount of the total coating layer was remained without being broken. Thus, the control of burst time was achieved by varying the coating amount.

### (Reference Example 1)

### Evaluation of intact CSA with a beagle dog model for diarrhea

As the main ingredient, used was compound A: methyl 1-(3,4-dimethoxyphenyl)-3-(3-ethylvaleryl)-4-hydroxy-6, 7, 8-trimethoxy-2-naphthoate.

The inhibitory effect of an intact CSA was examined by using male beagle dogs (n=5) wherein soft bowel movement was accompanied with an administration of a lactose trituration of compound A (dose: 30 mg/kg). To the dogs under starvation, were orally administered a lactose trituration of compound A (30 mg/kg as compound A) and intact CSA granules (DOWEX company, the grain of the dried product is about 500 µm, 100 mg/kg), each as being capsuled with 1/8 ounce capsules, then subjected to feed at 1 hr after the administration. As a result of time-lapse observation, 4 of 5 dogs had soft bowel movement or mucous stool by 8 hr after the administration, suggesting the inhibitory effect on the diarrhea was weak with intact CSA granules.

### (Experimental Example 1)

### Evaluation of enteric-coated CSA with a beagle dog model for diarrhea

The experiment was carried out by using male beagle dogs (n=5) wherein soft bowel movement was accompanied with an administration of a lactose trituration of compound A (dose: 30 mg/kg). To fasted-dogs, were orally administered a lactose trituration of compound A (30 mg/kg as compound A) and the enteric-coated CSA granules of Example 1 (69.4 mg/kg as CSA), each as being capsuled with 1/8 ounce capsules, then subjected to feed at 1 hr after the administration. As a result of time-lapse observation, 4 of 5 dogs had no soft bowel movement or mucous stool by 8 hr after the administration, suggesting a great inhibitory effect on the diarrhea.

### (Experimental Example 2)

### Evaluation of ethyl cellulose-coated CSA with a beagle dog model for diarrhea

The experiment was carried out by using male beagle (n=5) wherein soft bowel movement was accompanied with an administration of a lactose trituration of compound A (dose: 30 mg/kg). To fated-dogs, were orally administered a lactose trituration of compound A (30 mg/kg as compound A) and the cellulose-coated CSA granules of Example 2 (C: 74.6 mg/kg as CSA and D: 67.1 mg/kg as CSA), each as being capsuled with 1/8 ounce capsules, then subjected to feed at 1 hr after the administration. As a result of time-lapse observation, 4 of 5 dogs had no soft bowel movement or mucous stool by 8 hr after the administration in both cases of granules C and D, suggesting a great inhibitory effect on the diarrhea.

Further, the inhibitory effect on the diarrhea was examined with CSA granules D under a reduced dose of 20.1 mg/kg as CSA. As a result of time-lapse observation, 3 of 5 dogs had no soft bowel movement or mucous stool by 8 hr after the administration, suggesting a great inhibitory effect on the diarrhea.

### (Experimental Example 3)

### Evaluation of CSA capsuled in an enteric-coated capsule with a beagle dog model for diarrhea

The experiment was carried out by using male beagle dogs (n=5) wherein soft bowel movement was accompanied with an administration of a lactose trituration of compound A (dose: 30 mg/kg). To fasted-dogs, were orally administered a lactose trituration of compound A (30 mg/kg as compound A) and a CSA granule (DOWER company, the grain of the dried product is about 500 µm, 100 mg/kg) enteric-coated with HPMCAS-M (Shin-Etsu Chemical Co., Ltd.), each as being capsuled with 1/8 ounce capsules, then subjected to feed at 1 hr after the administration. As a result of time-lapse observation, 4 of 5 dogs had no soft bowel movement or mucous stool by 8 hr after the administration, suggesting a great inhibitory effect on the diarrhea.

### Industrial Applicability

The bile acid diarrhea can be effectively prevented by the present invention, thus providing a pharmaceutical composition, esp. antihyperlipidaemic agent, without a side effect like diarrhea.

## Claims

1. A preparation for preventing bile acid diarrhea, which comprises containing a bile acid adsorbent coated with a polymer so as to allow the release of the bile acid adsorbent around an area from the lower part of the small intestine to the cecum.

2. The preparation for preventing diarrhea according to claim 1, wherein the polymer is a water-insoluble polymer.

3. The preparation for preventing diarrhea according to claim 2, wherein the water-insoluble polymer is an enteric polymer or a pH-independent polymer.

4. The preparation for preventing diarrhea according to claim 3, wherein the enteric polymer is hydroxypropylmethylcellulose acetate succinate, hydroxypropylmethylcellulose phthalate or a methacrylic acid copolymer, and the pH-independent polymer is ethyl cellulose or an aminoalkylmethacrylic acid copolymer.

5. The preparation for preventing diarrhea according to claim 1, wherein an isolation layer is placed between the bile acid adsorbent and the polymer.

6. The preparation for preventing diarrhea according to claim 5, wherein the main ingredient of the isolation layer is phosphate or sulfate.

7. The preparation for preventing diarrhea according to claim 1, which is a granule.

8. A capsule which contains the granules of claim 7.

9. A preparation for preventing bile acid diarrhea, which comprises a capsule containing a bile acid adsorbent, wherein the surface of the capsule is coated with a polymer so as to allow the release of the bile acid adsorbent around an area from the lower part of the small intestine to the cecum .

10. A pharmaceutical composition, which comprises a combination of a bile acid re-absorption inhibitor and the preparation for preventing diarrhea of any one of claims 1 to 9.

11. The pharmaceutical composition according to claim 10, wherein the form of the bile acid re-absorption inhibitor is a granule, powder or a capsule.

12. The pharmaceutical composition according to claim 10, which comprises a combination of granules or powder, each containing the bile acid re-absorption inhibitor, and the preparation for preventing diarrhea of claim 7.

13. The pharmaceutical composition according to claim 10, which is a capsule containing the bile acid re-absorption inhibitor and the bile acid adsorbent, wherein the surface of the capsule is coated with a polymer so as to allow the release of the bile acid adsorbent around an area from the lower part of the small intestine to the cecum.

14. The pharmaceutical composition according to any one of claims 10 to 13, wherein the bile acid re-absorption inhibitor is absorbed in an area of the upper and middle parts of the small intestine.

15. The pharmaceutical composition according to claim 14, wherein the bile acid adsorbent is released around an area from the lower part of the small intestine to the cecum within several hours, after the absorption of the bile acid re-absorption inhibitor.

16. The pharmaceutical composition according to claim 14, wherein the bile acid adsorbent is released around an area from the lower part of the small intestine to the cecum at three to four fours after the absorbance of the bile acid re-absorption inhibitor.

17. The pharmaceutical composition according to claim 10 for use as an antihyperlipidaemic agent.

18. The pharmaceutical composition according to any one of claims 10 to 17, wherein the bile acid re-absorption inhibitor is methyl 1-(3,4-dimethoxyphenyl)-3-(3-ethylvaleryl)-4-hydroxy-6, 7, 8-trimethoxy-2-naphthoate or a glucuronic acid conjugate thereof.

19. Use of a bile acid adsorbent which is coated with a polymer so as to allow the release thereof, for preventing bile acid diarrhea upon oral administration of a bile acid re-absorption inhibitor.

20. Use according to claim 19, wherein the bile acid adsorbent is released around an area from the lower part of the small intestine to the cecum within several hours, after the absorption of the bile acid re-absorption inhibitor in an area of the upper and middle parts of the small intestine.

21. A method for preventing bile acid diarrhea upon oral administration of a bile acid re-absorption inhibitor, which comprises oral administration of the bile acid re-absorption inhibitor and a bile acid adsorbent coated with a polymer so as to allow the release of the bile acid adsorbent around an area from the lower part of the small intestine to the cecum, at the same time or a certain interval.

22. The method for preventing bile acid diarrhea according to claim 21, wherein the bile acid adsorbent is released around an area from the lower part of the small intestine to the cecum within several hours, after the absorbance of the bile acid re-absorption inhibitor in an area of the upper and middle parts of the small intestine.

23. A method for preventing or treating hyperlipemia, which comprises oral administration of the bile acid re-absorption inhibitor and a bile acid adsorbent coated with a polymer so as to allow the release of the bile acid adsorbent around an area from the lower part of the small intestine to the cecum, at the same time or a certain interval.
